(19) Europäisches Patentamt
European Patent Office
Office européen des brevets

(11) Veröffentlichungsnummer: **0 520 252 B1**

(12) **EUROPÄISCHE PATENTSCHRIFT**

(45) Veröffentlichungstag der Patentschrift: **27.09.95**

(51) Int. Cl.$^6$: **C07C 329/06**, A01N 47/06

(21) Anmeldenummer: **92109876.0**

(22) Anmeldetag: **12.06.92**

Die Akte enthält technische Angaben, die nach dem Eingang der Anmeldung eingereicht wurden und die nicht in dieser Patentschrift enthalten sind.

# (54) Acylierte Aminophenol-Derivate.

(30) Priorität: **25.06.91 DE 4120904**

(43) Veröffentlichungstag der Anmeldung:
**30.12.92 Patentblatt 92/53**

(45) Bekanntmachung des Hinweises auf die Patenterteilung:
**27.09.95 Patentblatt 95/39**

(84) Benannte Vertragsstaaten:
**BE CH DE FR GB IT LI NL**

(56) Entgegenhaltungen:
**EP-A- 0 125 901**
**EP-A- 0 293 718**

(73) Patentinhaber: **BAYER AG**
**D-51368 Leverkusen (DE)**

(72) Erfinder: **Krüger, Bernd-Wieland, Dr.**
**Unterboschbach 19**
**W-5060 Bergisch Gladbach 2 (DE)**
Erfinder: **Sasse, Klaus, Dr.**
**Pützweg 13**
**W-5060 Bergisch Gladbach 2 (DE)**
Erfinder: **Dehne, Heinz-Wilhelm, Dr.**
**Krischer Strasse 81**
**W-4019 Monheim (DE)**

**EP 0 520 252 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cycloalkylcarbonsäureanilide, ein Verfahren zu ihrer Herstellung und ihre Verwendung zur Bekämpfung von Schädlingen, insbesondere von Pilzen.

Es ist bekannt, daß bestimmte Carbamate gute fungizide Eigenschaften besitzen (vgl. EP 293 718).

Weiterhin sind viele Carbonsäureanilide mit fungizider Wirkung, insbesondere mit hoher Wirkung gegen Benzimidazol-tolerante Pflanzenpathogene, bekannt (vgl. EP 117 024, EP 125 901, EP 100 615).

Es wurden neue Cycloalkyl-carbonsäureanilide der allgemeinen Formel (I)

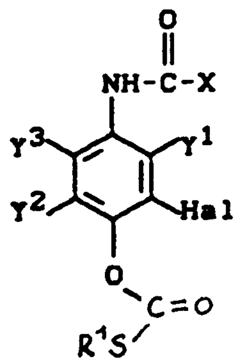

(I)

in welcher

| | |
|---|---|
| X | für gegebenenfalls Alkyl-substituiertes Cycloalkyl steht, |
| Hal | für Halogen steht und |
| $Y^1$, $Y^2$ und $Y^3$ | unabhängig voneinander für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen und |
| $R^1$ | für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl stehen, |

gefunden.

Die substituierten Cycloalkyl-carbonsäureanilide der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen substituierten Cycloalkyl-carbonsäureanilide der Formel (I)

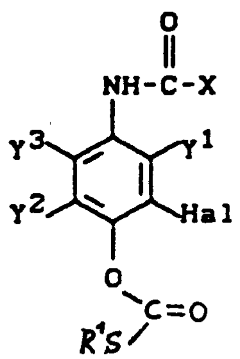

(I)

in welcher

| | |
|---|---|
| X | für gegebenenfalls Alkyl-substituiertes Cycloalkyl, |
| Hal | für Halogen und |
| $Y^1$, $Y^2$ und $Y^3$ | gleich oder verschieden sind und für Wasserstoff, Halogen, gegebenenfalls Halogen-substituiertes Alkyl, gegebenenfalls Halogen-substituiertes Alkoxy oder gegebenenfalls |

Halogen-substituiertes Alkylthio stehen und

$R^1$ für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl steht,

erhält, wenn man Aminophenole der Formel (II)

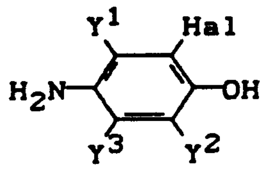

(II)

in welcher
Hal, $Y^1$, $Y^2$ und $Y^3$ die oben angegebenen Bedeutungen haben, in einem ersten Reaktionsschritt mit Carbonsäure-Derivaten der Formel (III)

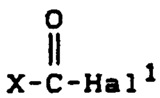

(III)

in welcher

X die oben angegebene Bedeutung hat und

$Hal^1$ für Halogen, vorzugsweise Chlor, oder eine bei Acylierungsreaktionen gebräuchliche Abgangsgruppe, vorzugsweise einen aktivierenden Esterrest, steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt diese erhaltenen Zwischenprodukte der Formel (IV)

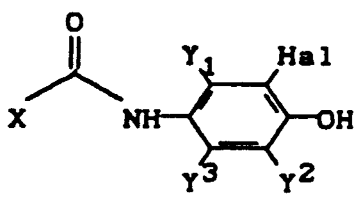

(IV)

in welcher
X, $Y^1$, $Y^2$, $Y^3$ und Hal die obengenannte Bedeutung haben,
mit Thiokohlensäure-Derivaten der Formel (V)

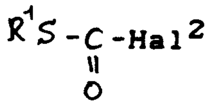

(V)

in welcher

$R^1$ die obengenannte Bedeutung hat und

$Hal^2$ für Halogen, vorzugsweise Chlor, oder eine bei Acylierungsreaktionen gebräuchliche Abgangsgruppe, vorzugsweise einen aktivierenden Esterrest, steht,

gegebenenfalls in Gegenwart eines Säureakzeptors und gegebenenfalls in Gegenwart eines Lösungs- oder Verdünnungsmittels umsetzt.

Schließlich wurde gefunden, daß die substituierten Cycloalkyl-carbonsäureanilideder Formel (I) u. a. eine hohe fungizide Wirksamkeit besitzen. Die neuen Verbindungen können auch mit anderen bekannten, hochwirksamen Verbindungen in synergistischen Mischungen verwendet werden.

Im Rahmen der vorliegenden Erfindung besitzen die Substituenten vorzugsweise die folgenden Bedeutungen:

Halogen kann, überall wo nicht anders angegeben, Fluor, Chlor, Brom und Iod, bevorzugt Fluor, Chlor und Brom bedeuten.

Alkyl, Alkoxy und Alkylthio stehen für einen Rest, der je Alkyleinheit 1 - 8, bevorzugt 1 - 6 und besonders bevorzugt 1 - 4 Kohlenstoffatome hat, z. B. Methyl, Ethyl, n- und iso-Propyl, n-, sec-, iso- und tert.-Butyl, Pentyl, n-Hexyl oder iso-Hexyl, Methoxy, Ethoxy, n- und iso-Propoxy, n-, sec,-, iso- und tert.-Butoxy, Pentoxy und Hexoxy, Methylthio, Ethylthio, n- und iso-Propylthio, n-, sec.-, iso- und tert.-Butylthio, Pentylthio und Hexylthio.

Halogenalkoxy bzw. Halogenalkylthio steht im allgemeinen für einen über Sauerstoff bzw. Schwefel gebundenen, geradkettigen oder verzweigten Kohlenwasserstoffrest mit 1 - 6 Kohlenstoffatomen und 1 - 9 gleichen oder verschiedenen Halogenatomen. Bevorzugt sind Reste mit 1 - 4 Kohlenstoffatomen und 1 - 5 gleichen oder verschiedenen Halogenatomen. Ganz besonders bevorzugt sind Reste mit 1 oder 2 Kohlenstoffatomen und 1 - 3 gleichen oder verschiedenen Halogenatomen. Beispielhaft seien genannt; Trifluormethoxy, Trichlormethoxy, Difluorchlormethoxy, Dichlorfluormethoxy, Difluorethoxy, Trifluorethoxy, Tetrafluorethoxy, Pentafluorethoxy, Trifluormethylthio, Trichlormethylthio, Difluorchlormethylthio, Dichlorfluormethylthio, Difluorethylthio, Trifluormethylthio, Tetrafluorethylthio.

Halogenalkyl hat die Bedeutung von Halogenalkoxy, mit dem Unterschied, daß das Sauerstoff bzw. Schwefelatom fehlt.

Cycloalkyl steht im allgemeinen für einen cyclischen Kohlenwasserstoffrest mit 3 - 10 Kohlenstoffatomen. Bevorzugt sind Reste mit 3 - 7 Kohlenstoffatomen. Beispielhaft seien genannt; Cyclopropyl, Cyclobutyl, Cyclopentyl, Cyclohexyl, Cycloheptyl und Cyclodecanyl.

Die Cycloalkylreste können ein- bis mehrfach substituiert sein. Als Substituenten seien Alkyl mit 1 - 4 Kohlenstoffatomen Halogen, Halogenalkyl mit 1 bis 4 Kohlenstoffatomen und 1 bis 9 gleichen oder verschiedenen Halogenatomen, Alkoxy mit 1 bis 4 Kohlenstoffatomen, Hydroxy und Alkylcarbonyloxy mit 1 bis 6 Kohlenstoffatomen im Alkylteil genannt.

Phenyl Phenylalkyl und substituiertes Phenoxyalkyl stehen im allgemeinen für Phenyl, Phenylalkyl und Phenoxyalkyl, in denen Phenylwasserstoffatome gegebenenfalls durch einen oder mehrere Substituenten $Y^{1'}$-$Y^{5'}$ersetzt sind. $Y^{1'}$-$Y^{5'}$ haben hierbei die Bedeutung von $Y^1$, $Y^2$ und $Y^3$ sowie Nitro und Cyano.

Alkenyl steht im allgemeinen für einen geradkettigen oder verzweigten Kohlenwasserstoffrest mit 2 bis 8 Kohlenstoffatomen und einer oder mehreren, bevorzugt mit einer oder zwei, Doppelbindungen. Bevorzugt ist Niederalkenyl mit 2 bis 6 Kohlenstoffatomen und einer Doppelbindung. Besonders bevorzugt ist ein Alkenylrest mit 2 bis 5 Kohlenstoffatomen und einer Doppelbindung.

Die erfindungsgemäßen substituierten Cycloalkyl-carbonsäureanilide sind durch die Formel (I) allgemein definiert. Bevorzugt sind Verbindungen der Formel (I), worin

X für gegebenenfalls ein- bis vierfach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht, wobei der Cycloalkylrest ein-bis sechsfach , gleich oder verschieden durch geradkettiges oder verzweigtes Alkyl mit 1 - 4 Kohlenstoffatomen substituiert sein kann,

Hal für Fluor, Chlor oder Brom steht,

$Y^1$, $Y^2$ und $Y^3$ gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 - 4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen, und

$R^1$ für gegebenenfalls einfach bis neunfach durch Halogen substituiertes $C_1$-$C_8$-Alkyl, $C_2$-$C_8$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl stehen oder für unsubstituiertes oder einfach bis fünffach, gleich oder verschieden durch $C_1$-$C_4$-Halogenalkyl, Halogen, $C_1$-$C_4$-Alkyl, $C_1$-$C_4$-Alkoxy, Hydroxy, $C_1$-$C_6$-Alkylcarbonyloxy substituiertes $C_3$-$C_7$-Cycloalkyl stehen oder für unsubstituiertes oder im Phenylteil einfach bis fünffach, gleich oder verschieden durch $Y^{1'}$-$Y^{5'}$ substituiertes Phenyl-$C_1$-$C_4$-alkyl oder für unsubstituiertes oder im Phenylteil einfach bis fünffach, gleich oder verschieden durch $Y^{1'}$-$Y^{5'}$ substituiertes Phenoxyalkyl steht, wobei $Y^{1'}$-$Y^{5'}$ die Bedeutung von $Y^1$-$Y^3$, $NO_2$ und Cyano haben.

Besonders bevorzugt sind Verbindungen der Formel (I), worin

X für ein- oder zweifach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht, und

$R^1$ für $C_1$-$C_6$-Alkyl, $C_2$-$C_6$-Alkenyl, $C_1$-$C_4$-Alkoxy-$C_1$-$C_4$-alkyl, $C_1$-$C_4$-Alxoxy-$C_1$-$C_4$-alkoxy-$C_1$-$C_4$-alkyl, $C_1$-$C_4$-Alkylthio-$C_1$-$C_2$-alkyl stehen oder für unsubstituiertes oder einfach bis dreifach, gleich oder verschieden durch Fluor, Chlor, Brom, $C_1$-$C_2$-Alkyl, $C_1$-$C_2$-Alkoxy, $C_1$-$C_6$-Alkylcarbonyloxy substituiertes $C_3$-$C_7$-Cycloalkyl stehen oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl-$C_1$-$C_2$-alkyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenoxy-$C_1$-$C_2$-alkyl steht.

Ganz besonders bevorzugt sind Verbindungen der Formel (I), worin

X für in 1-Stellung durch Methyl oder Ethyl substituiertes Cyclopentyl oder Cyclohexyl steht, welche gegebenenfalls durch einen weiteren Alkylrest mit 1 - 3 Kohlenstoffatomen zusätzlich substituiert sind,

Hal für Fluor, Chlor oder Brom steht und

$Y^1$, $Y^2$ und $Y^3$ gleich oder verschieden sind und für Wasserstoff, Fluor, Chlor, Brom oder Trifluormethyl stehen, und

$R^1$ für $C_1$-$C_4$-Alkyl, $C_2$-$C_5$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl stehen oder für einfach bis dreifach, gleich oder verschieden durch Methoxy, Fluor, Chlor, Brom, Methyl, $C_1$-$C_4$-Alkylcarbonyloxy substituiertes $C_3$-$C_7$-Cycloalkyl stehen oder für einfach bis dreifach, gleich oder verschieden durch Nitro, Cyano, Fluor, Chlor, Brom, Methyl, Methoxy substituiertes Phenyl oder für einfach bis dreifach, gleich oder verschieden durch Nitro, Cyano, Fluor, Chlor, Brom, Methyl, Methoxy substituiertes Phenylmethyl steht.

Verwendet man beispielsweise 2,6-Dichlor-4-amino-phenol, 1-Methyl-1-chlorcarbonylcyclohexan und Thiokohlensäure-s-butylester als Ausgangsstoffe, so kann der Reaktionsverlauf durch das folgende Formelschema wiedergegeben werden:

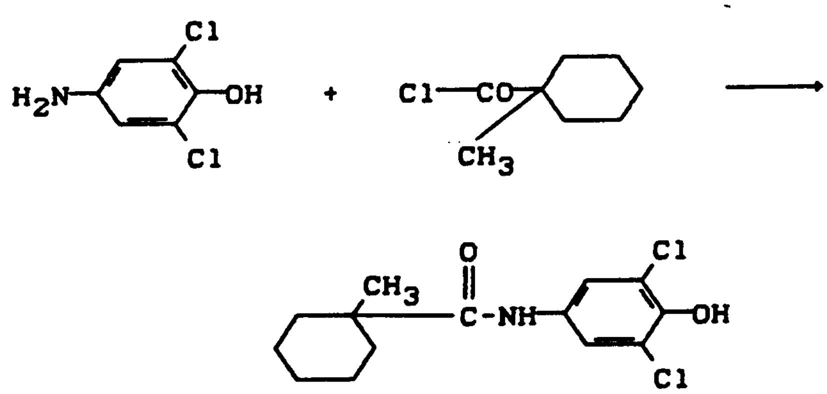

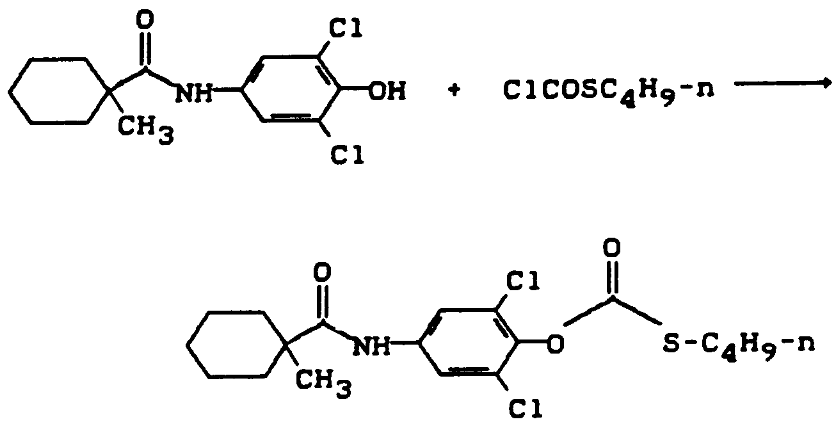

Die zur Durchführung des erfindungsgemäßen Verfahrens als Ausgangsstoffe benötigten Aminophenole sind durch die Formel (II) allgemein definiert. In dieser Formel (II) haben die Reste Hal und $Y^1$ - $Y^3$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar (vergl. "Methoden der organischen Chemie", Houben-Weyl, Band VI/1c, Phenole, Teil 1, Georg Thieme Verlag, Stuttgart, 1976, und "Reaktionen der organischen Synthese", Cesare Ferri, S. 81, 89, 91, 97, 118, 120, 122, 124, 126, 128, Georg Thieme Verlag, Stuttgart, 1978).

Die 4-Amino-2-chlor- bzw. -2-brom-6-trifluormethylphenole sind bekannt aus Jp. Kokai Tokkyo Koho Jp 61/126055 und z. B. 4-Amino-2,3,5,6-tetrafluorphenol aus Zh. Org. Khim. 10(9), 1923-1927 (1974). Die Verbindungen der Formel (II A)

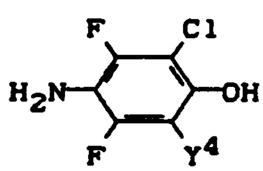

(II A)

in welcher

$Y^4$ für Fluor oder Chlor steht, sind Gegenstand von EP-A-293 718 und werden z.B. hergestellt aus entsprechenden Hydroxybenzoesäuren der Formel (VA)

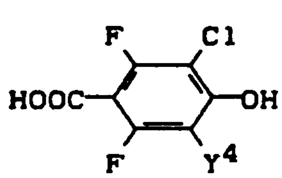

(VA)

durch Decarboxylierung mit anschließender Nitrierung der entstehenden Phenole der Formel (VI A)

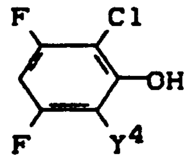

(VI A)

zu den Nitroverbindungen der Formel (VII A)

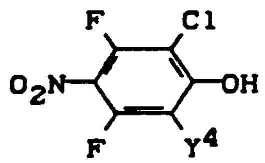

(VII A)

die dann hydriert werden, z. B. mit Wasserstoff und Raney-Nickel, zu den entsprechenden Aminen der Formel (II A).

Auch die Verbindungen der Formel (VII A) sind Gegenstand der EP-A-293 718 europäischen Anmeldung.

Die zur Durchführung des erfindungsgemäßen Verfahrens außerdem benötigten Cycloalkancarbonsäure-Derivate sind durch die Formel (III), in der X für Cycloalkyl steht, allgemein definiert In dieser Formel (III) haben die Reste X und $Hal^1$ die bereits im Zusammenhang mit der Beschreibung der erfindungsgemäßen Verbindungen der Formel (I) gegebenen Bedeutungen. Die Verbindungen sind bekannt und nach Analogieverfahren herstellbar (vergl. Diversi et. al., Synthesis 1971, 258; US 3 674 831; "Reaktionen der organischen Synthese" Cesare Ferri, S. 460, 461, 1978, Georg Thieme Verlag, Stuttgart); Houben-Weyl, Methoden der organischen Chemie, Bd. E5 Tl.1, S.211, 320, 343, 428 ff, G. Thieme-Verlag, Stuttgart, 1985).

Die zur Durchführung des erfindungsgemäßen Verfahrens außerdem benötigten Thio-Kohlensäure-Derivate der Formel (V), in welcher Z und $Hal^2$ die oben angegebene Bedeutung haben sind bekannt und nach Analogieverfahren herstellbar (vgl. Georg Thieme Verlag, Stuttgart; Houben-Weyl, "Methoden der organischen Chemie, Kohlensäure-Derivate, Bd. E4, S. 30 ff, Georg Thieme Verlag, Stuttgart, 1983).

Die bei dem erfindungsgemäßen Verfahren als Zwischenprodukte verwendeten Acylaminophenole sind durch die Formel (IV) definiert.

Das erfindungsgemäße Verfahren wird gegebenenfalls in Gegenwart von Säureakzeptoren durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische und heterocyclische Amine, beispielsweise Triethylamin, Trimethylamin, Dimethylanilin, 1,8-Diazabicyclo-(5,4,0)-undec-7-en, Dimethylbenzylamin und Pyridin.

Zur Durchführung des erfindungsgemäßen Verfahrens setzt man im ersten Reaktionsschritt vorzugsweise auf ein Mol Amino-Phenol der allgemeinen Formel (II) 1 - 2 Mol, insbesondere 1 - 1,4 Mol, der Verbindungen der allgemeinen Formel (III) ein.

Für den zweiten Reaktionsschritt des erfindungsgemäßen Verfahrens setzt man vorzugsweise auf 1 Mol Acylaminophenol der Formel (IV) 1-2 Mol, insbesondere 1-1,4 Mol, der Verbindungen der allgemeinen Formel (V) ein.

Als Verdünnungsmittel zur Durchführung des erfindungsgemäßen Verfahrens kommen praktisch alle inerten organischen Verdünnungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und - ethylester, Nitrile wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren wird im allgemeinen bei Temperaturen zwischen -50 °C und 120 °C durchgeführt. Bevorzugt wird der Bereich zwischen 0 °C und 110 °C. Die Umsetzungen werden im

allgemeinen bei Normaldruck durchgeführt.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillieren", d. h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z. B. durch Kristallisation. Zur Charakterisierung der Verbindungen dienen Brechungsindex, Schmelzpunkt, $R_f$-Wert oder Siedepunkt.

Die erfindungsgemäßen Wirkstoffe sind für den Gebrauch zur Bekämpfung von Schädlingen, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

## Herstellungsbeispiele

Herstellung der Ausgangsverbindungen:

### Beispiel A1:

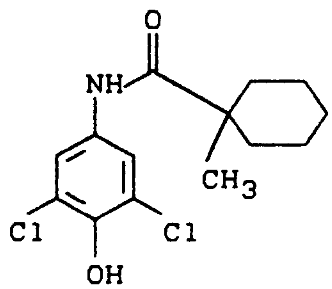

18,5 g (0,085 Mol) 4-Amino-2,6-dichlorphenol werden in 150 ml Tetrahydrofuran gelöst und zunächst mit 8,6 g (0,085 Mol) Triethylamin, dann bei 0 °C Innentemperatur mit 15 g (0,094 Mol) 1-Methylcyclohexancarbonsäurechlorid versetzt. Man rührt über Nacht bei 20 °C und fügt dann zur Vervollständigung der Reaktion erneut 5 g Carbonsäurechlorid und 2,8 g Triethylamin zur Reaktionsmischung. Nach 2 Stunden wird auf Eis gegossen und der abgesaugte Feststoff aus Toluol umkristallisiert. Man erhält die obengenannte Verbindung mit dem Schmelzpunkt 140 °C; Ausbeute; 22,3 g (= 87 % der Theorie).

Analog werden die Verbindungen der Formel (IV) bzw. (IVa) erhalten.

### Beispiel A2

3,5-Dichlor-2,6-difluor-4-hydroxybenzoesäure

In einer Rührapparatur werden 300 g Kaliumhydroxid, 600 ml Wasser, 15 g Tetrabutylammoniumchlorid und 135 g 3,5-Dichlor-2,4,6-trifluorbenzotrifluorid vorgelegt und dann für 5 Stunden am Rückfluß erhitzt. Nach Ende der Reaktion wird abgekühlt und durch Zutropfen von Salzsäure sauer gestellt. Das Festprodukt wird abgesaugt und im Vakuum getrocknet. Ausbeute: 93 g mit einem Schmelzpunkt 102 - 105 °C.

### Beispiel A3

3-Chlor-2,5,6-trifluor-4-hydroxy-benzoesäure

Analog Beispiel A1 werden aus 400 g NaOH, 1200 ml Wasser, 15 g Tetraethylammoniumchlorid und 276 g 3-Chlortetrafluorbenzotrifluorid bei 6-stündigem Erhitzen unter Rückfluß 238 g Produkt erhalten mit einem Schmelzpunkt von 87 - 90 °C.

Beispiel A4

2,6-Dichlor-3,5-difluorphenol

50 g 3,5-Dichlor-2,6-difluor-4-hydroxy-benzoesäure und 10 ml Dimethylformamid werden vermischt und erhitzt. Bei 105 - 130 °C entwickelt sich Kohlendioxid und man läßt bei dieser Temperatur ausreagieren. Anschließend rührt man 200 ml Toluol und danach 80 ml Wasser ein, trennt die Phasen, trocknet die organische Phase und destilliert anschließend. Man erhält 34 g des Produktes mit einem Siedepunkt von 87 - 88 °C und einem Brechungsindex von $n_D^{20}$ : 1,5310.

Beispiel A5

Analog Beispiel A3 erhält man 2-Chlor-3,5,6-trifluorphenol, mit einem Siedepunkt 68 - 70 °C / 20 mbar.

Beispiel A6

2,6-Dichlor-3,5-difluor-4-nitro-phenol

In 70 ml Essigsäure werden 20 g 2,6-Dichlor-3,5-difluorphenol vorgelegt und 8 g 98 %ige Salpetersäure zugetropft. Anschließend wird für 2 Stunden bei Raumtemperatur nachgerührt, in 150 ml Dichlormethan aufgenommen und zweimal mit Wasser gewaschen. Nach Abdestillieren des Dichlormethans verbleiben 18 g Produkt. Nach GC-Analyse 94 %ig.

Beispiel A7

2-Chlor-3,5,6-trifluor-4-nitrophenol

Analog Beispiel A5 werden durch Nitrierung aus 28 g 2-Chlor-3,5,6-trifluorphenol 25 g 2-Chlor-3,5,6-trifluor-4-nitrophenol erhalten mit einer Reinheit von 93 % und einem Schmelzpunkt von 107 - 109 °C.

Beispiel A8

2,6-Dichlor-3,5-difluor-4-amino-phenol

18 g 2,6-Dichlor-3,5-difluor-4-nitrophenol werden in 100 ml Methanol in Gegenwart von 1,5 g Raney-Nickel bei 25 - 45 °C mit 30 - 50 bar Wasserstoff bis zum Ende der Wasserstoffaufnahme hydriert. Nach Filtration wird die Lösung unter vermindertem Druck vom Lösungsmittel befreit. Es verbleiben 13 g Aminophenol (GC-Reinheit 98,4 %); Fp. 151 °C.

Beispiel A9

2-Chlor-3,5,6-trifluor-4-amino-phenol

Analog Beispiel A7 werden aus 25 g 2-Chlor-3,5,6-trifluor-4-nitro-phenol in 120 ml Methanol und 2 g Raney-Nickel durch Hydrierung 20 g Aminophenol (GC-Reinheit 97 %) erhalten.

Beispiel 1

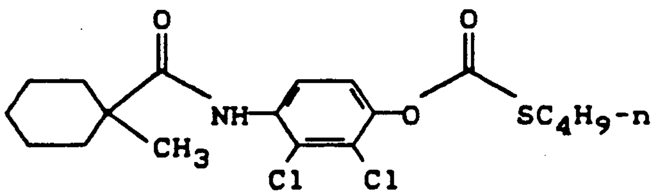

5 g (0,0165 mol) 4-(1-Methylcyclohexyl-carbonyl-2,3-dichlorphenol werden in 20 ml Tetrahydrofuran und 2,5 ml (0,019 mol) Triethylamin gelöst und bei 20 ° C 2,5 g (0,0177 mol) Thiokohlensäure-s-butylester-chlorid zugetropft. Man rührt eine Stunde bei 50 ° C, kühlt ab und gibt die Reaktionslösung auf Eiswasser. Der Feststoff wird abgesaugt und getrocknet.

Man erhält Thiokohlensäure-s-butylester-O-(2,3-dichlor-4-(1-methyl-cyclohexylcarbonyl))-phenylester mit einem Schmelzpunkt Fp. 62 ° C. Analog werden erhalten:

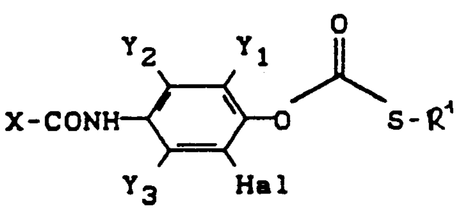

| Bsp.-Nr. | X | $Y_1$ | $Y_2$ | $Y_3$ | Hal | $R^1$ | phys. Daten (Fp °C) |
|---|---|---|---|---|---|---|---|
| 2 | 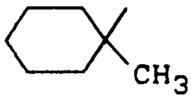 | H | H | Cl | Cl | $CH_2CH_2-C_4H_9-t$ | 66 |
| 3 | " | H | H | Cl | Cl | $CH(CH_3)C_2H_5$ | 103 |
| 4 | " | H | H | Cl | Cl | $CH_2-C_4H_9-t$ | 88 |
| 5 | " | H | H | Cl | Cl | $C_3H_7-i$ | 102 |
| 6 | " | H | H | Cl | Cl | $C_2H_5$ | 56 |
| 7 | " | H | H | Cl | Cl | $CH(CH_3)CH_2-C_3H_7-i$ | 77 |
| 8 | " | H | H | Cl | Cl | $CH_2$ 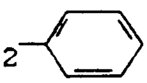 | 55 |
| 9 | " | H | H | Cl | Cl | $C_4H_9-t$ | 112 |
| 10 | " | H | H | Cl | Cl | $CH_2CH_2-C_3H_7-i$ | 68 |
| 11 | " | H | H | Cl | Cl | $CH_2CH_2OCH_3$ | |
| 12 | 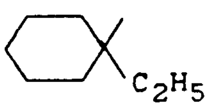 | H | H | Cl | Cl | $CH_3$ | |
| 13 | 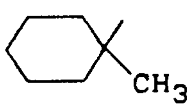 | H | Cl | H | Cl | $CH_3$ | |
| 14 | " | H | H | $CH_3$ | Cl | $CH_3$ | |

Beispiel

Botrytis-Test (Buschbohne) / protektiv

Lösungsmittel: 4,7 Gewichtsteile Aceton
Emulgator : 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß folgender Herstellungsbeispiele:

**Patentansprüche**

**1.** Cycloalkyl-carbonsäureanilide der allgemeinen Formel

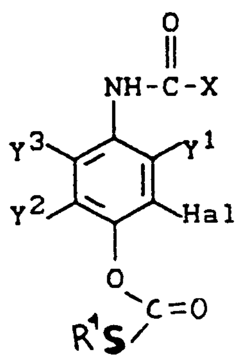

(I)

in welcher

| | |
|---|---|
| X | für gegebenenfalls Alkyl-substituiertes Cycloalkyl steht, |
| Hal | für Halogen steht und |
| $Y^1$, $Y^2$ und $Y^3$ | unabhängig voneinander für Wasserstoff, Halogen, gegebenenfalls Halogensubstituiertes Alkyl, gegebenenfalls Halogensubstituiertes Alkoxy oder gegebenenfalls Halogen-substituiertes Alkylthio stehen und |
| $R^1$ | für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl steht. |

**2.** Verbindungen der Formel (I) gemäß Anspruch 1, worin

| | |
|---|---|
| X | für gegebenenfalls ein- bis vierfach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht wobei der Cycloalkylrest ein- bis sechsfach, gleich oder verschieden durch geradkettiges oder verzweigtes Alkyl mit 1 - 4 Kohlenstoffatomen substituiert sein kann, |
| Hal | für Fluor, Chlor oder Brom steht, |
| $Y^1$, $Y^2$ und $Y^3$ | gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 -4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen, |
| $R^1$ | für gegebenenfalls einfach bis neunfach durch Halogen substituiertes $C_1$-$C_8$-Alkyl, $C_2$-$C_8$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl stehen oder für unsubstituiertes oder einfach bis fünffach, gleich oder verschieden durch $C_1$-$C_4$-Halogenalkyl, Halogen, $C_1$-$C_4$-Alkyl, $C_1$- |

$C_4$-Alkoxy, Hydroxy, $C_1$-$C_6$-Alkylcarbonyloxy substituiertes $C_3$-$C_7$-Cycloalkyl stehen oder für unsubstituiertes oder im Phenylteil einfach bis fünffach, gleich oder verschieden durch $Y^{1'}$-$Y^{5'}$ substituiertes Phenyl-$C_1$-$C_4$-alkyl oder für unsubstituiertes oder im Phenylteil einfach bis fünffach, gleich oder verschieden durch $Y^{1'}$-$Y^{5'}$ substituiertes Phenoxyalkyl steht,
wobei $Y^{1'}$-$Y^{5'}$ die Bedeutung von $Y^1$-$Y^3$, $NO_2$ und Cyano haben.

**3.** Verbindungen der Formel (I) gemäß Anspruch 1, worin

X für ein- oder zweifach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht, und

$R^1$ für $C_1$-$C_6$-Alkyl, $C_2$-$C_6$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_2$-alkyl stehen oder für unsubstituiertes oder einfach bis dreifach, gleich oder verschieden durch Fluor, Chlor, Brom, $C_1$-$C_2$-Alkyl, $C_1$-$C_2$-Alkoxy, $C_1$-$C_6$-Alkylcarbonyloxy substituiertes $C_3$-$C_7$-Cycloalkyl stehen oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenyl-$C_1$-$C_2$-alkyl oder für unsubstituiertes oder einfach bis dreifach, gleich oder verschieden durch Nitro, Cyano, Fluor, Chlor, Brom, Methyl, Trifluormethyl, Trifluormethylthio, Methoxy, Trifluormethoxy substituiertes Phenoxy-$C_1$-$C_2$-alkyl stehen.

**4.** Verbindungen der Formel (I) gemäß Anspruch 1, worin

X für in 1-Stellung durch Methyl oder Ethyl substituiertes Cyclopentyl oder Cyclohexyl steht, welche gegebenenfalls durch einen weiteren Alkylrest mit 1 - 3 Kohlenstoffatomen zusätzlich substituiert sind,

Hal für Fluor, Chlor oder Brom steht und

$Y^1$, $Y^2$ und $Y^3$ gleich oder verschieden sind und für Wasserstoff, Fluor, Chlor, Brom oder Trifluormethyl stehen, und

$R^1$ für $C_1$-$C_4$-Alkyl, $C_2$-$C_5$-Alkenyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl stehen oder für einfach bis dreifach, gleich oder verschieden durch Methoxy, Fluor, Chlor, Brom, Methyl, $C_1$-$C_4$-Alkylcarbonyloxy substituiertes $C_3$-$C_7$-Cycloalkyl stehen oder für einfach bis dreifach, gleich oder verschieden durch Nitro, Cyano, Fluor, Chlor, Brom, Methyl, Methoxy substituiertes Phenyl oder für einfach bis dreifach, gleich oder verschieden durch Nitro, Cyano, Fluor, Chlor, Brom, Methyl, Methoxy substituiertes Phenylmethyl steht.

**5.** Verfahren zur Herstellung von substituierten Cycloalkylcarbonsaureaniliden der Formel (I)

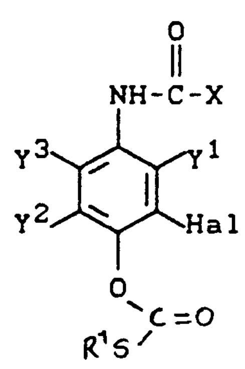

(I)

in welcher

X für gegebenenfalls Alkyl-substituiertes Cycloalkyl

Hal für Halogen und

$Y^1$, $Y^2$ und $Y^3$ gleich oder verschieden sind und für Wasserstoff, Halogen, gegebenenfalls Halogen-substituiertes Alkyl, gegebenenfalls Halogen-substituiertes Alkoxy oder gegebenenfalls Halogen-substituiertes Alkylthio stehen und

$R^1$ für gegebenenfalls durch Halogen substituiertes Alkyl, Alkenyl, Alkoxyalkyl, Alkylthioalkyl, Polyalkoxyalkyl und gegebenenfalls substituiertes Cycloalkyl, gegebenenfalls substituiertes Phenyl, gegebenenfalls substituiertes Phenylalkyl oder substituiertes Phenoxyalkyl steht,

dadurch gekennzeichnet, daß man Aminophenole der Formel (II)

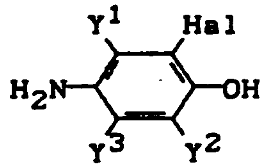

(II)

in welcher

Hal, $Y^1$, $Y^2$ und $Y^3$ die oben angegebenen Bedeutungen haben, in einem ersten Reaktionsschritt mit Carbonsäure-Derivaten der Formel (III)

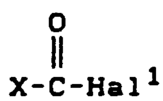

(III)

in welcher

X die oben angegebene Bedeutung hat und

$Hal^1$ für Halogen, oder eine bei Acylierungsreaktionen gebräuchliche Abgangsgruppe steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt diese erhaltenen Zwischenprodukte der Formel (IV)

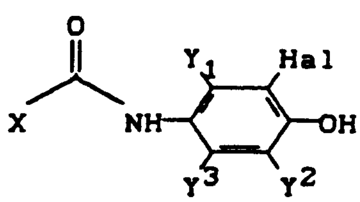

(IV)

in welcher

X, $Y^1$, $Y^2$, $Y^3$ und Hal die obengenannte Bedeutung haben,

mit Thiokohlensäure-Derivaten der Formel (V)

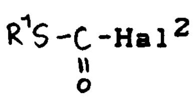

(V)

in welcher

$R^1$ die obengenannte Bedeutung hat und

$Hal^2$ für Halogen oder eine bei Acylierungsreaktionen gebräuchliche Abgangsgruppe steht,

gegebenenfalls in Gegenwart eines Säureakzeptors und gegebenenfalls in Gegenwart eines Lösungs-

oder Verdünnungsmittels umsetzt.

**6.** Schädlingsbekämpfungsmittel, gekennzeichnet durch einen Gehalt an mindestens einem Cycloalkylcarbonsäureanilid der Formel (I) nach den Ansprüchen 1 bis 4.

**7.** Verwendung von Cycloalkylcarbonsäureanilid der Formel (I) nach den Ansprüchen 1 bis 4 zur Bekämpfung von Schädlingen.

**8.** Verfahren zur Bekämpfung von Schädlingen, dadurch gekennzeichnet, daß man Cycloalkyl carbonsäureanilid der Formel (I) nach den Ansprüchen 1 bis 4 auf Schädlinge und/oder ihren Lebensraum einwirken läßt.

**9.** Verfahren zur Herstellung von Schädlingsbekämpfungsmitteln, dadurch gekennzeichnet, daß man Cycloalkylcarbonsäureanilid der Formel (I) nach den Ansprüchen 1 bis 4 mit Streckmitteln und/oder oberflächenaktiven Mitteln vermischt.

**Claims**

**1.** Cycloalkyl-carboxanilides of the general formula

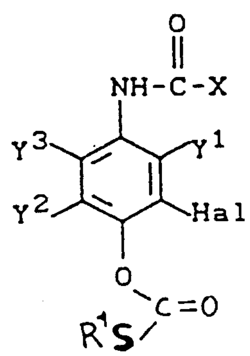

(I)

in which

| | |
|---|---|
| X | represents optionally alkyl-substituted cycloalkyl, |
| Hal | represents halogen and |
| $Y^1$, $Y^2$ and $Y^3$ | independently of one another represent hydrogen, halogen, optionally halogen-substituted alkyl, optionally halogen-substituted alkoxy or optionally halogen-substituted alky lthio and |
| $R^1$ | represents optionally halogen-substituted alkyl, alkenyl, alkoxyalkyl, alkylthioalkyl, polyalkoxyalkyl and optionally substituted cycloalkyl, optionally substituted phenyl, optionally substituted phenylalkyl or substituted phenoxyalkyl. |

**2.** Compounds of the formula (I) according to Claim 1, in which

| | |
|---|---|
| X | represents cyclopropyl, cyclobutyl, cyclopentyl, cyclohexyl or cycloheptyl, each of which is optionally monosubstituted to tetrasubstituted by identical or different straight-chain or branched alkyl substitutents having 1 - 4 carbon atoms, it being possible for the cycloalkyl radical to be monosubstituted to hexasubstituted by identical or different straight-chain or branched alkyl substituents having 1 - 4 carbon atoms, |
| Hal | represents fluorine, chlorine or bromine, |
| $Y^1$, | $Y^2$and $Y^3$ are identical or different and represent hydrogen, fluorine, chlorine, bromine, straight-chain or branched alkyl having 1 - 4 carbon atoms, straight-chain or branched alkoxy or alkylthio having in each case 1 - 4 carbon atoms, or represent halogenoalkyl, halogenoalkoxy or halogenoalkylthio, each of which has 1 - 4 carbon atoms in the straight-chain or branched alkyl moiety and 1 - 5 identical or different halogen atoms, |
| $R^1$ | represents $C_1$-$C_8$-alkyl, $C_2$-$C_8$-alkenyl, $C_1$-$C_4$-alkoxy-$C_1$-$C_4$-alkyl, $C_1$-$C_4$-alkoxy-$C_1$-$C_4$-alkoxy-$C_1$-$C_4$-alkyl, $C_1$-$C_4$-alkylthio-$C_1$-$C_4$-alkyl, each of which is optionally monosubstituted to |

nonasubstituted by halogen, or represents $C_3$-$C_7$-cycloalkyl which is unsubstituted or monosubstituted to pentasubstituted by identical or different substituents from the series comprising $C_1$-$C_4$-halogenoalkyl, halogen, $C_1$-$C_4$-alkyl, $C_1$-$C_4$-alkoxy, hydroxyl or $C_1$-$C_6$-alkylcarbonyloxy, or represents phenyl-$C_1$-$C_4$-alkyl which is unsubstituted or monosubstituted to pentasubstituted in the phenyl moiety by identical or different $Y^{1'}$-$Y^{5'}$ substituents, or represents phenoxyalkyl which is unsubstituted or monosubstituted to pentasubstituted in the phenyl moiety by identical or different $Y^{1'}$-$Y^{5'}$ substituents,
where $Y^{1'}$-$Y^{5'}$ have the meaning of $Y^1$-$Y^3$, $NO_2$ and cyano.

**3.** Compounds of the formula (I) according to Claim 1, in which

X represents cyclopropyl, cyclobutyl, cyclopentyl, cyclohexyl or cycloheptyl, each of which is monosubstituted or disubstituted by identical or different straight-chain or branched alkyl substituents having 1 - 4 carbon atoms,

$Y^1$, $Y^2$ and $Y^3$ are identical or different and represent hydrogen, fluorine, chlorine, bromine, methyl or trifluoromethyl and

Hal represents fluorine, chlorine or bromine, and $R^1$ represents $C_1$-$C_6$-alkyl, $C_2$-$C_6$-alkenyl, $C_1$-$C_4$-alkoxy-$C_1$-$C_4$-alkyl, $C_1$-$C_4$-alkoxy-$C_1$-$C_4$-alkoxy-$C_1$-$C_4$-alkyl, $C_1$-$C_4$-alkylthio-$C_1$-$C_2$-alkyl, or represents $C_3$-$C_7$-cycloalkyl, each of which is unsubstituted or monosubstituted to trisubstituted by identical or different substituents from the series comprising fluorine, chlorine, bromine, $C_1$-$C_2$-alkyl, $C_1$-$C_2$-alkoxy or $C_1$-$C_6$-alkylcarbonyloxy, or represents phenyl which is unsubstituted or monosubstituted to trisubstituted by identical or different substituents from the series comprising nitro, cyano, fluorine, chlorine, bromine, methyl, trifluoromethyl, trifluoromethylthio, methoxy or trifluoromethoxy, or represents phenyl-$C_1$-$C_2$-alkyl which is unsubstituted or monosubstituted to trisubstituted by identical or different substituents from the series comprising nitro, cyano, fluorine, chlorine, bromine, methyl, trifluoromethyl, trifluoromethylthio, methoxy or trifluoromethoxy, or represents phenoxy-$C_1$-$C_2$-alkyl which is unsubstituted or monosubstituted to trisubstituted by identical or different substituents from the series comprising nitro, cyano, fluorine, chlorine, bromine, methyl, trifluoromethyl, trifluoromethylthio, methoxy or trifluoromethoxy.

**4.** Compounds of the formula (I) according to Claim 1, in which

X represents cyclopentyl or cyclohexyl, each of which is substituted in the 1-position by methyl or ethyl and each of which is optionally additionally substituted by a further alkyl radical having 1 - 3 carbon atoms,

Hal represents fluorine, chlorine or bromine and

$Y^1$, $Y^2$ and $Y^3$ are identical or different and represent hydrogen, fluorine, chlorine, bromine or trifluoromethyl, and

$R^1$ represents $C_1$-$C_4$-alkyl, $C_2$-$C_5$-alkenyl, $C_1$-$C_4$-alkoxy-$C_1$-$C_4$-alkyl, $C_1$-$C_4$-alkoxy-$C_1$-$C_4$-alkoxy-$C_1$-$C_4$-alkyl, or represents $C_3$-$C_7$-cycloalkyl which is monosubstituted to trisubstituted by identical or different substituents from the series comprising methoxy, fluorine, chlorine, bromine, methyl or $C_1$-$C_4$-alkylcarbonyloxy, or represents phenyl which is monosubstituted to trisubstituted by identical or different substituents from the series comprising nitro, cyano, fluorine, chlorine, bromine, methyl or methoxy, or represents phenylmethyl which is monosubstituted to trisubstituted by identical or different substituents from the series comprising nitro, cyano, fluorine, chlorine, bromine, methyl or methoxy.

**5.** Process for the preparation of substituted cycloalkylcarboxanilides of the formula (I)

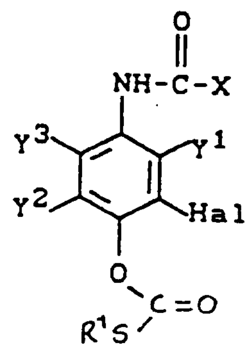

(I)

in which

X represents optionally alkyl-substituted cycloalkyl,

Hal represents halogen and

$Y^1$, $Y^2$ and $Y^3$ are identical or different and represent hydrogen, halogen, optionally halogen-substituted alkyl, optionally halogen-substituted alkoxy or optionally halogen-substituted alkylthio and

$R^1$ represents optionally halogen-substituted alkyl, alkenyl, alkoxyalkyl, alkylthioalkyl, polyalkoxyalkyl and optionally substituted cycloalkyl, optionally substituted phenyl, optionally substituted phenylalkyl or substituted phenoxyalkyl,

characterised in that aminophenols of the formula (II)

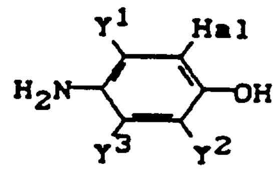

(II)

in which

Hal, $Y^1$, $Y^2$ and $Y^3$ have the abovementioned meanings, are reacted, in a first reaction step, with carboxylic acid derivatives of the formula (III)

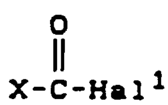

(III)

in which

X has the abovementioned meaning and

$Hal^1$ represents halogen, or a leaving group customary in the case of acylation reactions,

if appropriate in the presence of an acid acceptor and if appropriate in the presence of a solvent or diluent, and these intermediates obtained, of the formula (IV)

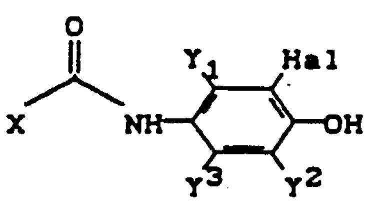

(IV)

in which X, $Y^1$, $Y^2$, $Y^3$ and Hal have the abovementioned meaning, are then reacted, in a second reaction step, with thiocarbonic acid derivatives of the formula (V)

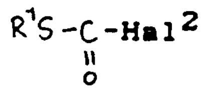

(V)

in which

$R^1$ has the abovementioned meaning and

$Hal^2$ represents halogen, or a leaving group customary in the case of acylation reactions,

if appropriate in the presence of an acid acceptor and if appropriate in the presence of a solvent or diluent.

**6.** Pesticides, characterised in that they contain at least one cycloalkylcarboxanilide of the formula (I) according to Claims 1 to 4.

**7.** Use of cycloalkylcarboxanilide of the formula (I) according to Claims 1 to 4 for combating pests.

**8.** Method of combating pests, characterised in that cycloalkylcarboxanilide of the formula (I) according to Claims 1 to 4 is allowed to act on pests and/or their environment.

**9.** Process for the preparation of pesticides, characterised in that cycloalkylcarboxanilide of the formula (I) according to Claims 1 to 4 is mixed with extenders and/or surface-active agents.

**Revendications**

**1.** Cycloalkyl-carboxanylide de formule générale

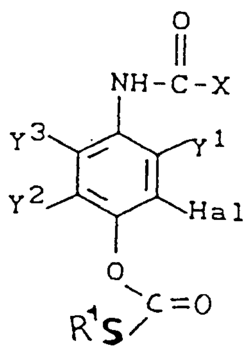

(I)

dans laquelle

X est un groupe cycloalkyle portant éventuellement un substituant alkyle,

Hal représente un halogène et

$Y^1$, $Y^2$ et $Y^3$ représentent, indépendamment les uns des autres, de l'hydrogène, un halogène, un groupe alkyle éventuellement substitué par un halogène, un groupe alkoxy éventuellement substitué par un halogène ou un groupe alkylthio éventuellement substitué par un halogène et

$R^1$ est un groupe alkyle, alcényle, alkoxyalkyle, alkylthioalkyle, polyalkoxyalkyle, éventuellement substitué par un halogène et un groupe cycloalkyle éventuellement substitué par un halogène, un groupe phényle éventuellement substitué par un halogène, un groupe phénylalkyle éventuellement substitué par un halogène ou un groupe phénoxyalkyle éventuellement substitué par un halogène.

**2.** Composés de formule (I) suivant la revendication 1, dans lesquels

X est un groupe cyclopropyle, cyclobutyle, cyclopentyle, cyclohexyle ou cycloheptyle portant le cas échéant 1 à 4 substituants, identiques ou différents, alkyle linéaires ou

ramifiés ayant 1 à 4 atomes de carbone, le reste cycloalkyle pouvant porter un à six substituants, identiques ou différents, alkyle linéaires ou ramifiés ayant 1 à 4 atomes de carbone,

Hal — représente du fluor, du chlore, du brome,

$Y^1$, $Y^2$ et $Y^3$ — sont identiques ou différents et représentent de l'hydrogène, du fluor, du chlore, du brome, un groupe alkyle linéaire ou ramifié ayant 1 à 4 atomes de carbone, un groupe alkoxy ou alkylthio linéaire ou ramifié ayant chacun 1 à 4 atomes de carbone, un groupe halogénalkyle, halogénalkoxy ou halogénalkylthio ayant chacun 1 à 4 atomes de carbone dans la partie alkyle linéaire ou ramifiée et portant un à cinq atomes d'halogène identiques ou différents,

$R^1$ — est un groupe alkyle en $C_1$ à $C_8$, alcényle en $C_2$ à $C_8$, (alkoxy en $C_1$ à $C_4$)-(alkyle en $C_1$ à $C_4$), (alkoxy en $C_1$ à $C_4$)-(alkoxy en $C_1$ à $C_4$)-(alkyle en $C_1$ à $C_4$), (alkylthio en $C_1$ à $C_4$)-(alkyle en $C_1$ à $C_4$), portant le cas échéant un à neuf substituants halogéno ou un groupe cycloalkyle en $C_3$ à $C_7$ non substitué ou portant 1 à 5 substituants, identiques ou différents, halogénalkyle en $C_1$ à $C_4$, halogéno, alkyle en $C_1$ à $C_4$, alkoxy en $C_1$ à $C_4$, hydroxy, (alkyle en $C_1$ à $C_6$)carbonyloxy, ou un groupe phényl-(alkyle en $C_1$ à $C_4$) non substitué ou portant dans la partie phényle un à cinq substituants $Y^{1'}$-$Y^{5'}$ identiques ou différents, $Y^{1'}$-$Y^{5'}$ correspondant à la définition de $Y^1$-$Y^3$, $NO_2$ et cyano.

**3.** Composés de formule (I) suivant la revendication 1, dans lesquels

X — est un groupe cyclopropyle, cyclobutyle, cyclopentyle, cyclohexyle ou cycloheptyle portant un ou deux substituants, identiques ou différents, alkyle linéaire ou ramifié ayant 1 à 4 atomes de carbone,

$Y^1$, $Y^2$ et $Y^3$ — sont identiques ou différents et représentent l'hydrogène, le fluor, le chlore, le brome, un groupe méthyle ou trifluorométhyle et

Hal — représente le fluor, le chlore ou le brome, et

$R^1$ — est un groupe alkyle en $C_1$ à $C_6$, alcényle en $C_2$ à $C_6$, (alkoxy en $C_1$ à $C_4$)-(alkyle en $C_1$ à $C_4$), (alkoxy en $C_1$ à $C_4$)-(alkoxy en $C_1$ à $C_4$)-(alkyle en $C_1$ à $C_4$), (alkylthio en $C_1$ à $C_4$)-(alkyle en $C_1$ à $C_2$) ou un groupe cycloalkyle en $C_3$ à $C_7$ non substitué ou portant un à trois substituants, identiques ou différents, fluoro, chloro, bromo, alkyle en $C_1$ ou $C_2$, alkoxy en $C_1$ ou $C_2$ (alkyle en $C_1$ à $C_6$)carbonyloxy, ou un groupe phényle non substitué ou portant un à trois substituants, identiques ou différents, nitro, cyano, fluoro, chloro, bromo, méthyle, trifluorométhyle, trifluorométhylthio, méthoxy, trifluorométhoxy ou un groupe phényl-(alkyle en $C_1$ ou $C_2$) non substitué ou portant un à trois substituants, identiques ou différents, nitro, cyano, fluoro, chloro, bromo, méthyle, trifluorométhyle, trifluorométhylthio, méthoxy, trifluorométhoxy ou un groupe phénoxy-(alkyle en $C_1$ ou $C_2$) non substitué ou portant un à trois substituants, identiques ou différents, nitro, cyano, fluoro, chloro, bromo, méthyle, trifluorométhyle, trifluorométhylthio, méthoxy, trifluorométhoxy.

**4.** Composés de formule (I) suivant la revendication 1, dans lesquels

X — est un groupe cyclopentyle ou cyclohexyle portant en position 1 un substituant méthyle ou éthyle, et substitué en outre le cas échéant par un autre reste alkyle ayant 1 à 3 atomes de carbone,

Hal — représente le fluor, le chlore ou le brome, et

$Y^1$, $Y^2$ et $Y^3$ — sont identiques ou différents et représentent le fluor, le chlore, le brome ou un groupe trifluorométhyle, et

$R^1$ — est un groupe alkyle en $C_1$ à $C_4$, alcényle en $C_2$ à $C_5$, (alkoxy en $C_1$ à $C_4$)-(alkyle en $C_1$ à $C_4$), (alkoxy en $C_1$ à $C_4$)-(alkoxy en $C_1$ à $C_4$)-(alkyle en $C_1$ à $C_4$) ou un groupe cycloalkyle en $C_3$ à $C_7$ portant un à trois substituants, identiques ou différents, méthoxy, fluoro, chloro, bromo, méthyle, (alkyle en $C_1$ à $C_4$)carbonyloxy ou un groupe phényle portant un à trois substituants, identiques ou différents, nitro, cyano, fluoro, chloro, bromo, méthyle, méthoxy, ou un groupe phénylméthyle portant un à trois substituants, identiques ou différents, nitro, cyano, fluoro, chloro, bromo, méthyle, méthoxy.

**5.** Procédé de production de cycloalkylcarboxanylides substitués de formule (I)

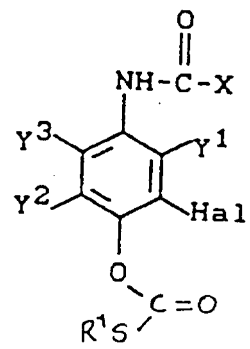

(I)

dans laquelle

X est un groupe cycloalkyle portant éventuellement un substituant alkyle,

Hal est un halogène et

$Y^1$, $Y^2$ et $Y^3$ sont identiques ou différents et représentent l'hydrogène, un halogène, un groupe alkyle éventuellement substitué par un halogène, un groupe alkoxy éventuellement substitué par un halogène ou un groupe alkylthio éventuellement substitué par un halogène et

$R^1$ est un groupe alkyle, alcényle, alkoxyalkyle, alkylthioalkyle, polyalkoxyalkyle éventuellement substitué par un halogène et un groupe cycloalkyle éventuellement substitué par un halogène, un groupe phényle éventuellement substitué par un halogène, un groupe phénylalkyle éventuellement substitué par un halogène ou un groupe phénoxyalkyle éventuellement substitué par un halogène,

caractérisés en ce qu'on fait réagir des aminophénols de formule (II)

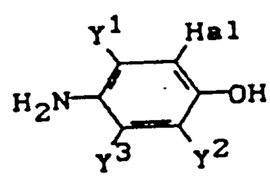

(II)

dans laquelle

Hal, $Y^1$, $Y^2$ et $Y^3$ ont les définitions données ci-dessus, dans une première étape réactionnelle avec des dérivés d'acides carboxyliques de formule (III)

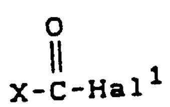

(III)

dans laquelle

X a la définition indiquée ci-dessus et

$Hal^1$ est un halogène ou un groupe partant d'emploi classique dans des réactions d'acylation,

le cas échéant en présence d'un accepteur d'acide et en la présence éventuelle d'un solvant ou d'un diluant, puis on fait réagir dans une seconde étape réactionnelle les produits intermédiaires ainsi obtenus de formule (IV)

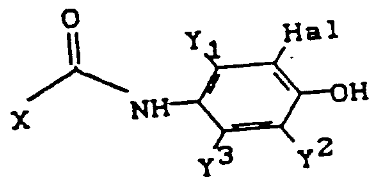

(IV)

dans laquelle
X, $Y^1$, $Y^2$, $Y^3$ et Hal ont la définition indiquée ci-dessus, avec des dérivés d'acides thiocarboniques de formule (V)

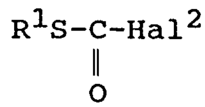

(V)

dans laquelle

- $R^1$ a la définition indiquée ci-dessus et
- $Hal^2$ représente un halogène ou un groupe alkyle partant d'emploi classique dans des réactions d'acylation,

le cas échéant en présence d'un accepteur d'acide et en la présence éventuelle d'un solvant ou d'un diluant.

**6.** Compositions pesticides, caractérisées par une teneur en au moins un cycloalkylcarboxanylide de formule (I) suivant les revendications 1 à 4.

**7.** Utilisation d'un cycloalkylcarboxanylide de formule (I) suivant les revendications 1 à 4 pour combattre des parasites.

**8.** Procédé pour combattre des parasites, caractérisé en ce qu'on fait réagir un cycloalkylcarboxanylide de formule (I) suivant les revendications 1 à 4 sur des parasites et/ou sur leur milieu.

**9.** Procédé de préparation de compositions de pesticides, caractérisé en ce qu'on mélange un cycloalkylcarboxanylide de formule (I) suivant les revendications 1 à 4 avec des diluants et/ou des agents tensioactifs.